# FASCICULE DE BREVET EUROPEEN

(11) **EP 3 593 132 B1**
(45) Date de publication et mention de la délivrance du brevet: **04.05.2022**
(21) Numéro de dépôt: 18712967.1
(22) Date de dépôt: 07.03.2018
(51) Int. Cl.: G01N 33/48

(54) **SUPPORT POUR UN ÉCHANTILLON D'ESPÈCES BIOLOGIQUES**
TRÄGER FÜR EINE PROBE EINER BIOLOGISCHEN SPEZIES
SUPPORT FOR A BIOLOGICAL SPECIES SAMPLE

(30) Priorité: 08.03.2017 FR 1751890
(43) Date de publication de la demande: 15.01.2020
(73) Titulaire: UNIVERSITE GRENOBLE ALPES, 38400 Saint Martin d'Hères (FR); Centre Hospitalier Universitaire de Grenoble, 38700 La Tronche (FR); Medimprint, 38700 La Tronche (FR); Etablissements Cattin, 38610 Giéres (FR)
(72) Inventeur: DREYFUS, Matthieu, 38000 Grenoble (FR); ROSSIGNOLO, Philippe, 38130 Echirolles (FR); ZACCARIA, Affif, 38100 Grenoble (FR)
(74) Mandataire: Bronchart, Quentin
(86) Numéro de dépôt international: PCT/FR2018/050523
(87) Numéro de publication internationale: WO 2018/162847

(56) Documents cités:
- WO-A1-2013/105095
- WO-A1-2014/141262
- FR-A1- 2 999 872
- US-A1- 2005 220 677
- US-A1- 2007 207 554

## Description

### DOMAINE DE L'INVENTION

La présente invention concerne un support pour un échantillon d'espèces biologiques.

### ARRIERE PLAN TECHNOLOGIQUE DE L'INVENTION

On connait de l'état de la technique des dispositifs permettant de prélever sur un tissu ou sur un fluide, par simple mise en contact d'une surface de capture, un échantillon d'espèces biologiques en vue de son analyse.

Les espèces biologiques adsorbées sur la surface de capture peuvent comprendre des cellules, des bactéries, des virus. Il peut s'agir de molécules, ou de macro molécules telles que des protéines ou des peptides. Les dimensions de ces espèces biologiques peuvent être très variables, de l'ordre du nanomètre jusqu'à plusieurs dizaines de microns. Les analyses menées sur l'échantillon prélevé peuvent être une analyse par microscopie confocale ou par spectroscopie de masse avec désorption laser MALDI (acronyme de l'expression anglo-saxonne « Matrix Assisted Laser Desorption/Ionization » ou SALDI (acronyme de l'expression anglo-saxonne « Surface Assisted Laser Desorption/Ionization »). Les analyses biochimiques, de biologie moléculaire et polyomiques (protéomique, transcriptomique et génomique, etc.) sont aussi possibles. Il peut également s'agir de conserver et de mettre en culture les cellules prélevées.

On a représenté sur la figure 1 un tel dispositif de prélèvement d'espèces biologiques, connu par exemple des documents WO2013/098703, FR2955024, WO2006/090220. Le dispositif 1 est formé d'une tige de préhension 2 et d'un élément de capture d'espèces biologiques 3. L'élément de capture 3 est assemblé à la tige de préhension 2, par exemple par l'intermédiaire d'une colle biocompatible. La tige de préhension 2 présente une longueur adaptée à l'application visée, c'est-à-dire à la profondeur d'insertion nécessaire pour atteindre les tissus ou l'organe dont on souhaite faire l'analyse. Cette longueur peut être par exemple de l'ordre de 20 cm. La tige présente également une section dont la dimension la plus grande est relativement étroite, de l'ordre du millimètre ou inférieure à 1 mm. La surface libre de l'élément de capture 3 constitue la surface de capture 3a qui reçoit l'échantillon prélevé.

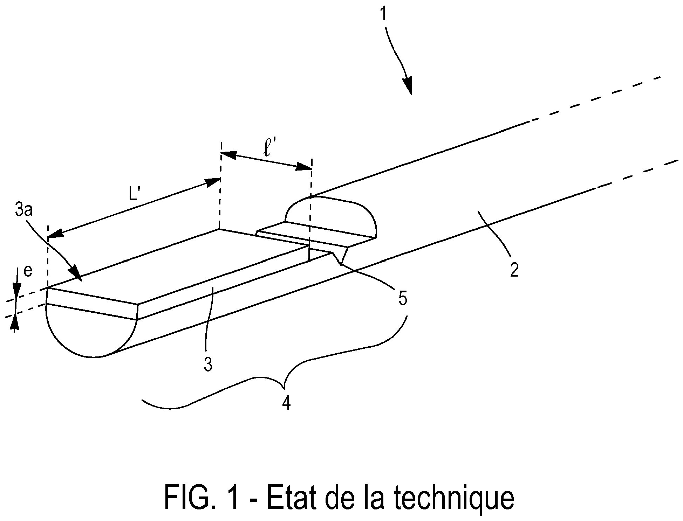

La surface de capture 3a est une surface structurée et/ou fonctionnalisée encourageant l'adsorption des espèces biologiques lorsque cette surface est mise en contact avec les tissus ou le fluide dont on souhaite faire l'analyse. L'élément 3 et la surface 3a de capture peuvent être en silicium, un élément de capture 3 peut ainsi être une puce de silicium. L'élément de capture présente une longueur « L' »qui peut s'étendre sur plusieurs centimètres, une largeur « l' » de l'ordre du mm, et une épaisseur « e » de quelques dixièmes de mm, ainsi que cela est indiqué sur la figure 1. La tige de préhension 2 peut être munie d'une pluralité d'éléments de capture 3, par exemple juxtaposés les uns aux autres selon la longueur de la tige 2.

On désignera dans la présente demande par l'expression « portion distale » 4, l'extrémité de la tige de préhension 2 comprenant l'assemblage de l'élément de capture 3 et de cette tige.

La tige de préhension 2 peut être munie d'une amorce mécanique de rupture 5 ou d'un autre mécanisme permettant de séparer la portion distale 4 du reste de la tige 2. Celle-ci en effet est encombrante et pourrait gêner la manipulation, le traitement de l'échantillon ou sa mise en place dans un équipement d'analyse.

Après prélèvement, et afin de faciliter la manipulation de l'échantillon prélevé, l'élément de capture 3 ou la portion distale 4 du dispositif 1 est généralement disposé sur un support. Ce support peut permettre de conserver l'échantillon ou le préparer à l'analyse.

On connaît du document FR3007634 un support pouvant accueillir un échantillon d'espèces biologiques prélevé à l'aide d'un dispositif conforme à celui qui vient d'être présenté. Ce support est formé d'un corps présentant une surface plane sur laquelle une pluralité de logements a été formée. Les logements présentent des dimensions adaptées pour recevoir la portion distale 4 du dispositif de prélèvement et exposer la surface de capture 3 afin de permettre l'analyse de l'échantillon par un équipement d'analyse.

L'utilisation d'un tel support présente toutefois des limitations. Elle nécessite de préalablement séparer la partie distale 4 du dispositif 1 pour la placer ensuite manuellement dans son logement. Cette manipulation n'est pas aisée à réaliser, sans prendre le risque de détériorer ou de contaminer l'échantillon.

De plus, une fois placée dans son logement, la portion distale 4 n'est pas maintenue fixe sur le support. Elle est donc susceptible d'en être délogée, par exemple lors du déplacement du support vers l'équipement d'analyse. Cet inconvénient est encore plus marqué lorsque le support est conçu pour recevoir une pluralité d'éléments de capture 3.

On connaît également du document WO 2006/090220 un support présentant, sur un de ses côtés, un évidement longitudinal permettant d'insérer dans le support la portion distale 4 du dispositif de prélèvement 1. L'évidement dans lequel est placé l'échantillon communique avec la surface du support par l'intermédiaire de canaux débouchant à cette surface. Ce support permet de mettre en contact la surface de capture 3a enfouie dans le support, avec un liquide en vue de séparer et prélever les espèces biologiques de la surface de capture 3a. Lorsque la partie distale 4 du dispositif de prélèvement 1 est insérée dans le support, la surface de capture n'est pas exposée à la surface du support, ce qui ne permet pas de mener des analyses de type microscopie ou spectroscopie.

On connaît également du document US2007207554, un support présentant, sur un de ses côtés, un évidemment longitudinal permettant d'insérer dans le support une languette de test. Dans ce document, l'évidemment communique directement à la surface et comprend sur sa partie basse, une partie transparente permettant de faire des analyses de réfractométrie en faisant passer la lumière à travers l'échantillon.

La présente invention vise à pallier tout ou partie des inconvénients précités. Elle vise notamment à proposer un support pour un échantillon d'espèces biologiques simple d'utilisation, permettant de maintenir fixement l'élément de capture portant l'échantillon et de procéder à son analyse et/ou à son traitement.

### BREVE DESCRIPTION DE L'INVENTION

En vue de la réalisation de l'un au moins de ces buts, l'objet de l'invention propose un support pour un échantillon d'espèces biologiques recueilli sur un dispositif de prélèvement comprenant :
- une tige de préhension ;
- un élément de capture présentant une surface de capture pour recevoir l'échantillon, l'élément de capture étant assemblé à la tige de préhension pour former une portion distale du dispositif de prélèvement.

Le support présente une face principale plane délimitée par au moins un côté et un évidemment débouchant sur le côté du support dans lequel peut être inséré le dispositif de capture, l'évidemment consistant en une rainure formée sur la face principale du support pour recevoir la portion distale du dispositif de prélèvement, la rainure étant configurée pour coopérer avec la partie distale afin de la maintenir fixement sur ou dans le support.

Selon l'invention, le support comprend un organe de fixation d'une paroi sur sa face principale pour former un réservoir au-dessus d'une partie au moins de la rainure.

Selon d'autres caractéristiques avantageuses et non limitatives de l'invention, prises seules ou selon toute combinaison techniquement réalisable :
- la rainure présente une largeur mesurée au niveau de la face principale du support strictement inférieure à sa largeur maximale selon sa profondeur de manière à maintenir la partie distale du dispositif de prélèvement sur ou dans le support lorsque celui-ci est logé dans la rainure ;
- le support présente une pluralité de rainures ;
- les rainures sont parallèles entre elles ;
- le support est constitué d'un matériau transparent ;
- le support est muni sur sa face principale d'une couche conductrice ;
- Le support est constitué d'une plaque métallique ;
- la profondeur de la rainure est telle que la surface de capture affleure la face principale du support lorsque la portion distale du dispositif de prélèvement est logée dans la rainure ;
- les dimensions de la rainure sont ajustées aux dimensions de la portion distale pour former une liaison étanche ;

Selon un autre aspect, l'objet de l'invention propose un kit de traitement d'un échantillon d'espèces biologiques comprenant un support et une paroi d'un réservoir.

Selon d'autres caractéristiques avantageuses et non limitatives de ce kit, prises seules ou selon toute combinaison techniquement réalisable :
- le kit de traitement comprend de plus un capot amovible du réservoir ;
- la paroi du réservoir est formée d'une matière opaque ;
- le réservoir comprend une pluralité de compartiments étanches ;
- la paroi du réservoir comprend une encoche interne pour recevoir l'extrémité d'une micro pipette de dispense.

L'objet de l'invention propose également un système d'insertion d'un dispositif de prélèvement dans un support, comprenant :
- un premier élément pour placer fixement un support, dans un plan d'assemblage;
- un second élément pour placer fixement dans le plan d'assemblage un dispositif de prélèvement, le premier élément et le deuxième élément étant disposé l'un par rapport à l'autre pour que la tige de préhension soit placée en vis-à-vis et aligné à la rainure du support ;
- un dispositif d'entraînement pour déplacer le premier élément vis-à-vis du second élément pour les rapprocher l'un de l'autre et loger la portion distale du dispositif de prélèvement dans la rainure.

Le système d'insertion peut comprendre un dispositif de coupe pour désolidariser la portion distale du reste de la tige de prévention.

### BREVE DESCRIPTION DES DESSINS

D'autres caractéristiques et avantages de l'invention ressortiront de la description détaillée de l'invention qui va suivre en référence aux figures annexées sur lesquels :
- La figure 1 représente un dispositif de prélèvement d'un échantillon d'espèces biologique de l'état de la technique ;
- Les figures 2a et 2b représentent un support conforme à l'invention ;
- les figures 2c et 2d représentent deux exemples d'une coupe du support selon un axe perpendiculaire à la rainure, présentant la section d'une rainure d'un support conforme à l'invention ;
- La figure 3a représente un support muni d'un réservoir d'un liquide.
- La figure 3b représente un support conforme à l'invention comportant une entaille permettant de placer une paroi d'un réservoir.
- La figure 3c représente un réservoir pour un support conforme à l'invention.
- La figure 4 représente un système d'insertion d'un dispositif de prélèvement dans un support conforme à l'invention.

### DESCRIPTION DETAILLEE DE L'INVENTION

Par souci de simplification de la description à venir, les mêmes références sont utilisées pour des éléments identiques ou assurant la même fonction dans l'état de la technique ou dans les différents modes de mise en oeuvre exposés du procédé.

L'invention porte sur un support 6 pour un échantillon d'espèces biologiques. L'échantillon est recueilli sur une surface de capture 3a d'un dispositif de prélèvement 1 tel que décrit en introduction de la demande et dont a déjà présenté un exemple de réalisation en figure 1. Pour mémoire, on rappelle néanmoins que le dispositif 1 comprend une tige de préhension 2 et un élément de capture d'espèces biologiques 3 assemblé à une extrémité de la tige 2. Cet assemblage forme une portion distale 4 du dispositif de prélèvement 1. Cette portion distale 4 peut être sécable ou détachable. La tige de préhension peut donc prévoir une zone de rupture 5, comme cela est représenté sur la figure 1, ou plus généralement, un organe 5 permettant de désolidariser la portion distale 4 du reste du dispositif 1.

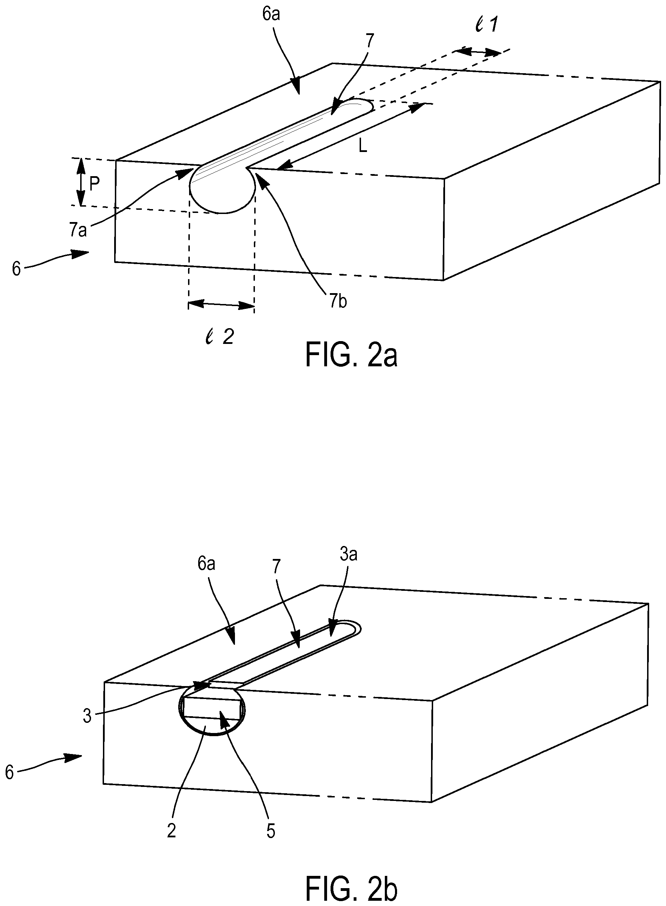

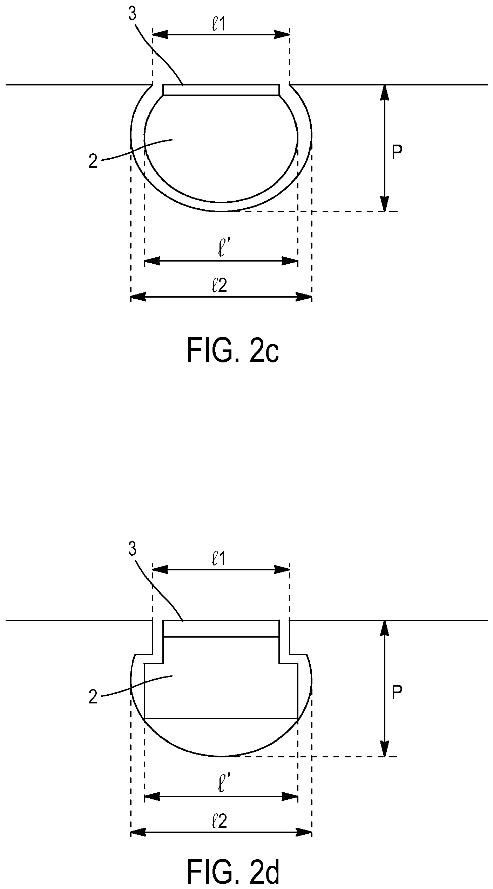

La figure 2a représente un support 6 conforme à l'invention. Le support 6 présente une face principale plane 6a, délimitée par au moins un côté ou un flanc. Sur l'exemple de la figure 2, le support 6 est de forme parallélépipédique, et présente donc quatre flancs latéraux formant les côtés de la face principale 6a. L'invention n'est toutefois pas limitée à cette forme, et le support peut présenter un nombre quelconque de flancs latéraux, définissant la face principale 6a.

Le support 6 présente un évidemment débouchant sur le côté du support dans lequel peut être inséré l'extrémité du dispositif de capture 1. Selon l'invention, cet évidemment consiste en une rainure 7 formée sur la face principale 6a du support 6, et s'étendant jusqu'au côté délimitant cette face principale 6a. Lorsque la partie distale 4 du dispositif de prélèvement est placée convenablement dans la rainure 7, la surface de capture 3 est exposée au niveau de la face principale 6a du support 6, comme cela est bien visible sur la figure 2b.

Selon l'invention, la rainure 7 est configurée pour coopérer avec la partie distale 4 du dispositif de prélèvement 1 afin de la maintenir fixement sur ou dans le support 6, c'est-à-dire que la partie distale 4 ne peut s'échapper de la rainure 7.

En d'autres termes, la rainure 7 comporte une partie en saillie 7a, 7b avec laquelle la portion distale 4 du dispositif est susceptible d'entrer en contact de sorte qu'elle ne puisse s'échapper de la rainure 7.

De manière particulièrement avantageuse, la partie en saillie 7a, 7b bloque également le mouvement de rotation de la portion distale 4 dans la rainure 7, autour de l'axe formé par la tige de préhension 2 ou de la partie restante de cette tige 2. On garantit ainsi que la surface de capture 3a reste bien parallèle ou coplanaire à la face principale 6a du support 6, ce qui contribue à la qualité de l'analyse.

D'une manière générale, la forme et les dimensions de la rainure 7 sont choisies pour correspondre à la forme et aux dimensions de la portion distale 4 du dispositif de prélèvement 1 afin de la maintenir fixement sur ou dans le support 6. Ces dimensions doivent être suffisamment grandes pour pouvoir y insérer la portion distale 4 du dispositif 1, en la glissant dans la rainure 7. Ces dimensions doivent être dans le même temps suffisamment ajustées pour que la portion distale 4 soit maintenue fixement dans la rainure 7.

De multiples configurations sont possibles pour assurer le maintien suffisant sur ou dans le support 6, et les figures 2c et 2d représentent, à titre d'illustration, deux exemples particuliers de rainure 7 et de dispositif 1 conformes à l'invention. Ces figures sont des vues en coupe du support 7 selon un plan perpendiculaire à la rainure 7. On a porté sur ces figures la longueur L, la profondeur P et la largeur l1, l2 de la rainure 7.

Dans ces deux exemples, la rainure 7 présente une largeur l1 mesurée au niveau de la face principale 6a du support 6 strictement inférieure à sa largeur maximale l2 selon sa profondeur. Dans le même temps, la largeur maximale l' de la portion distale 4 est supérieure à la largeur l1 de la rainure mesurée au niveau de la surface principale 6a. De la sorte, lorsque la partie distale 4 du dispositif 1 est logée de la rainure 7, elle est maintenue fixement dans la rainure 7 et ne peut s'en échapper.

Dans les deux exemples représentés, le disque engendré par la rotation de la section de la portion distale, le long d'un axe défini par la longueur de la tige présente une dimension plus importante que la section de la rainure. En conséquence, le mouvement de rotation de la portion distale 4 selon cet axe de rotation est bien bloqué.

Revenant à la description de la figure 2a et 2b, la longueur L de la rainure 7 est, de préférence, supérieure à la longueur L' de l'élément de capture 3. Ainsi, lorsque la partie distale 4 est placée en butée dans la rainure 7 l'élément de capture 2 est entièrement logé dans la rainure 7, c'est-à-dire qu'il ne déborde pas du support 6. De manière très avantageuse, la longueur L de la rainure 7 correspond ou est légèrement inférieure à la distance séparant l'extrémité de la tige de préhension 2, en butée dans la rainure, et l'amorce mécanique de rupture 5 ou l'organe de désassemblage 5 de la portion distale 4 de la tige 2.

Une fois le dispositif 1 inséré en butée dans la rainure 7, il est possible de rompre la tige de préhension 2 au niveau de l'amorce de rupture 5 et en détacher la portion distale 4 qui reste alors entièrement logée dans le support 6. Cette rupture peut être obtenue par application d'efforts sur la partie émergente de la tige de préhension 2, ces efforts pouvant, par exemple, être dirigés selon une direction perpendiculaire à la face principale 6a du support 6.

Avantageusement, et comme cela est bien visible sur les vues en coupe des figures 2c et 2d, la profondeur p de la rainure 7 est choisie de sorte que la surface de capture 2a soit affleurante à la face principale 6a du support 6. Cette configuration assure une bonne précision des analyses menées sur l'échantillon, notamment lorsque celles-ci correspondent à de la microscopie confocale. Par « affleurant », on signifie que la différence de niveau entre la surface de capture 3a et la face principale 6a du support 6 est inférieure à 50 microns, ou à 20 microns, ou à 10 microns. De préférence, cette différence de niveau est telle que la surface de capture 2a est positionnée légèrement sous la face principale 6a du support 6, de manière à éviter tout risque d'endommagement de la surface de capture 2a.

Dans ce mode de réalisation, il est également particulièrement avantageux de disposer d'une rainure 7 dont la largeur l1 mesurée au niveau de la face principale 6a du support 6 soit juste supérieure à la largeur l' de la surface de capture. Par exemple, la largeur l1 de la rainure 7 peut être supérieure de 10 microns à la largeur l' de l'élément de capture 3. De la sorte, un liquide dispensé à la surface du support 6 ne peut s'écouler facilement dans l'interstice existant entre la surface de capture 3a affleurante et la face principale 6a du support 6. On forme de la sorte une liaison étanche entre le support 6 et la portion distale 4a insérée dans la rainure 7. D'une manière plus générale, les dimensions de la rainure 7 peuvent être ajustées aux dimensions de la portion distale 4 pour former une liaison étanche. C'est notamment le cas lorsque le liquide présente une viscosité proche de celle l'eau.

Bien entendu, le support 6 présente une épaisseur suffisante pour pouvoir y former la rainure 7 sans constituer une zone pouvant rendre le support mécaniquement fragile. Ainsi, le support peut présenter une épaisseur supérieure à 2 fois ou supérieure à 5 fois à la profondeur de la rainure.

D'une manière générale, le support est constitué d'un matériau ou d'une pluralité de matériaux qui n'est pas susceptible de retenir les espèces biologiques afin d'éviter de contaminer les échantillons.

Dans certains cas, et selon les besoins de l'analyse qui doit être menée sur l'échantillon, le support 6 peut être choisi pour être constitué ou comprendre un matériau électriquement conducteur, ou être recouvert d'un film électriquement conducteur. Dans certains cas également, le matériau du support 6 peut être transparent. Dans le cas où un film conducteur est placé sur un tel support transparent, ce film peut être choisi en oxyde d'indium-étain (aussi connu par l'acronyme ITO de l'expression anglo-saxonne Indium Tin Oxyde) .

Ainsi, dans un exemple particulier de mise en oeuvre, le support 6 prend la forme d'une lame de matériau transparent (par exemple en verre ou PMMA) dont les dimensions normalisées permettent de facilement placer le support et l'échantillon dans l'équipement d'analyse.

Dans un second exemple particulier, le support 6 prend la forme d'une plaque métallique rectangulaire, par exemple en inox, dont les dimensions sont choisies pour être placées dans un équipement d'analyse, par exemple de spectroscopie de masse.

De manière avantageuse, le support 6 est muni d'une pluralité de rainures 7, conformes à celle qui vient d'être décrite. On peut de la sorte disposer sur un même support 6 une pluralité d'échantillons. Préférentiellement, ces rainures 7 sont disposées parallèles les unes aux autres de manière à faciliter le séquencement des analyses pour chaque échantillon dans l'équipement, sans avoir à excessivement manipuler le support 6.

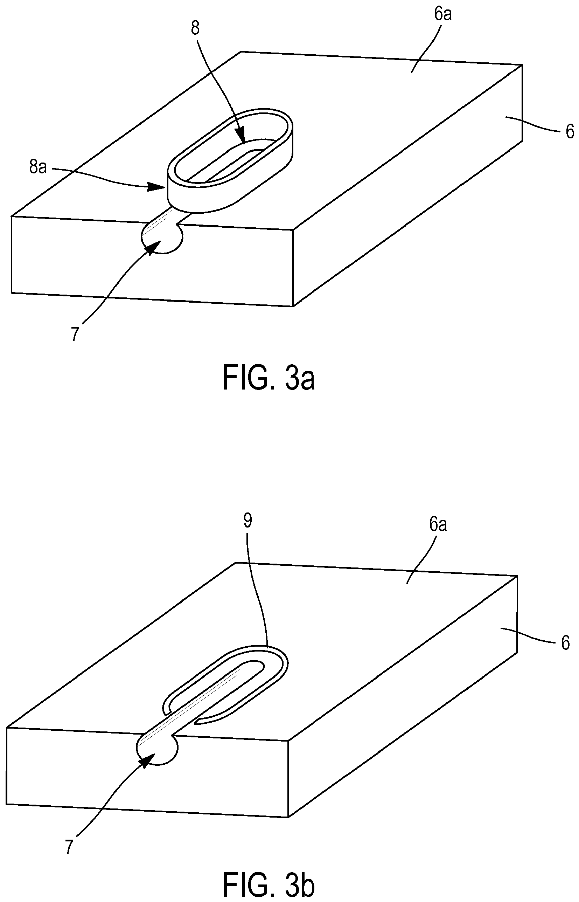

Dans le mode de réalisation préférée de l'invention, dans lequel le support 6 et la portion distale 4 forment une liaison étanche, le support 6 peut être muni d'un réservoir 8, disposé au-dessus d'une partie au moins de la rainure 7, comme cela est représenté sur la figure 3a. Le réservoir peut être constitué ou comprendre une paroi 8a présentant un contour fermé. Lorsque la paroi 8a est placée sur le support 6, la face principale 6a contribue à former le fond du réservoir 8. Le réservoir 8 est prévu pour contenir un liquide ou une solution de traitement tel qu'un tampon de lyse et mettre ce liquide en contact avec l'échantillon.

Le contour de la paroi 8a peut présenter une dimension, au niveau de sa face en contact avec le support 6, correspondant à la dimension (en largeur et/ou en longueur) de la rainure 7. Il est donc possible de mettre en contact l'échantillon et le liquide du réservoir 8, tout en limitant la quantité de liquide contenue dans le réservoir 8. Lorsqu'il s'agit de récupérer dans le liquide, les espèces biologiques de l'échantillon adsorbées sur la surface de capture 3a, le faible volume du réservoir 8 assure une concentration importante de ces espèces, ce qui est particulièrement avantageux.

Pour permettre de disposer fixement et de manière étanche la paroi 8a sur le support 6, celui-ci peut être muni d'un organe de fixation adapté. Par exemple, et comme cela est représenté schématiquement sur la figure 3b, la face principale 6a du support 6 peut être munie d'une entaille périphérique 9 ou d'une pluralité d'entailles périphérique à la rainure 7 permettant d'emboiter de manière démontable la partie inférieure de la paroi 8a du réservoir 8.

Le support 6 et le la paroi 8a du réservoir 8 peuvent être fournis dans un kit de traitement d'un échantillon d'espèces biologiques, pour permettre à un utilisateur de former le réservoir 8 selon son besoin.

La paroi 8a du réservoir 8 peut être opaque afin que la lumière n'affecte pas les réactions ou l'absence de réaction se produisant entre le liquide et les espèces biologiques. Pour les mêmes raisons, le réservoir 8 peut être muni d'un capot ou d'un couvercle qui peut être également opaque.

Tout comme le support 6, le réservoir 8 est réalisé dans un matériau qui ne retient pas ou qui n'est pas susceptible de retenir les espèces biologiques. Il peut par exemple s'agir de polyétheréthercétone ou de polypropylène.

Pour faciliter la dispense du liquide dans le réservoir 8, la paroi 8a peut être munie au moins d'une encoche interne conçue pour recevoir l'extrémité du micro pipette de dispense.

De manière avantageuse, le réservoir 8 peut comprendre au moins une paroi interne 8b permettant de définir, à l'intérieur du réservoir 8, une pluralité de compartiments internes et étanches entre eux.

On peut de la sorte procéder au traitement de différentes sections de la surface de capture 3a à l'aide de liquides dispensés dans chacun des compartiments. Ces liquides peuvent être identiques ou distincts entre eux. Les différentes sections de la surface de capture 3a peuvent avoir été en contact avec différents types de tissus, et donc porter des échantillons de natures différentes. On peut grâce au réservoir 8 à compartiments procéder au traitement simultané de ces sections.

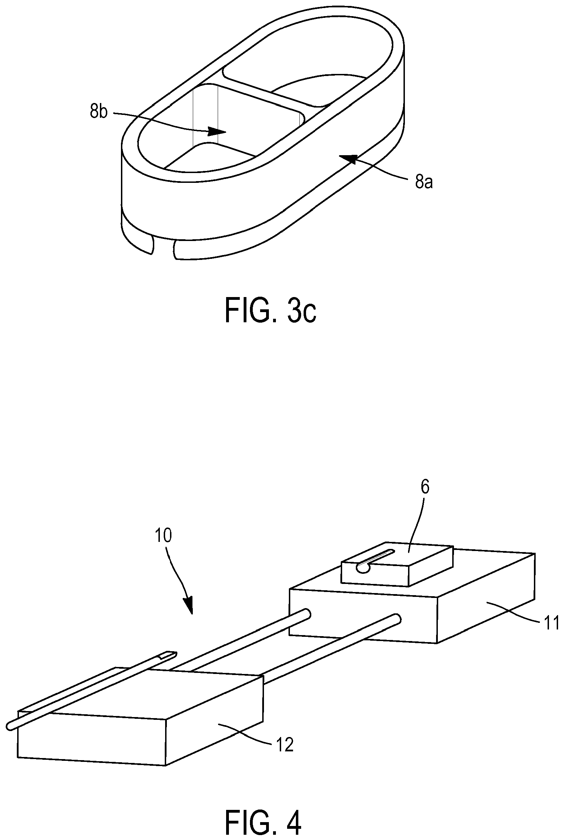

Par exemple, et comme cela est représenté sur la figure 3c, les parois internes 8b peuvent être agencées selon une direction essentiellement perpendiculaire à la rainure 7, lorsque le réservoir 8 est monté sur le support 6, afin de définir des compartiments du réservoir respectivement en surplomb d'une pluralité de sections contiguës de la surface de capture 3a, et successivement disposées dans la longueur de la rainure 7.

Selon un autre exemple, les parois internes 8b du réservoir 8 peuvent être agencées selon une direction essentiellement parallèle à la rainure 7 et définir au moins deux compartiments du réservoir 8 respectivement en surplomb d'une pluralité de sections de la surface de capture 3a contiguës dans la largeur de la rainure. Comme dans l'exemple précédent, on peut traiter ces deux sections, simultanément ou non, et donc éviter un prélèvement complémentaire.

Pour faciliter l'insertion du dispositif de prélèvements 1 dans le support 6 une fois le prélèvement réalisé, l'invention propose également un système 10 d'insertion du dispositif de prélèvement 1 dans le support 6. Un exemple d'un tel système est représenté sur la figure 4. Le système comprend un premier élément 11 pour maintenir fixement dans un plan d'assemblage le support 6. Il comprend également un second élément 12 pour placer fixement un dispositif de prélèvement 1 dans le même plan d'assemblage. Le premier élément 11 et le deuxième élément 12 sont disposés l'un par rapport à l'autre de sorte que la tige de préhension 2 soit placée en vis-à-vis et alignée à la rainure 7 du support 6. Le système d'insertion 10 comprend également un dispositif d'entraînement permettant de déplacer le premier élément 11 (et donc le support 6) vis-à-vis du second élément dompte 12 (et donc du dispositif de prélèvement 1) pour les rapprocher l'un de l'autre et loger la portion distale 4 du dispositif de prélèvement 1 dans la rainure 7.

Sur la figure 4 ce système d'insertion 11 comprend deux rails coulissants dans des ouvertures formées dans le premier élément 11, mais tout autre dispositif permettant de déplacer en translation le premier élément 11 vis-à-vis du second élément 12 avec suffisamment de précision peut convenir.

Le système 10 peut également comprendre un dispositif de découpe ou de désassemblage, par exemple du type guillotine, permettant de désolidariser la portion distale 4 du reste dispositif de prélèvement 1, par l'exemple au niveau de l'amorce de rupture mécanique 5 lorsqu'une telle amorce a été formée sur la tige de préhension 2 du dispositif de prélèvement.

Bien entendu l'invention n'est pas limitée aux modes de mise en oeuvre décrits et on peut y apporter des variantes de réalisation sans sortir du cadre de l'invention tel que défini par les revendications.

Ainsi, bien que l'on ait prévu d'associer le support 6 avec un réservoir 8, il est tout à fait possible d'associer ce support 6 avec un système micro fluidique disposé à la surface du support 6, et notamment en surplomb de la rainure 7 de manière, par exemple, à contrôler avec une grande précision la quantité de liquide ou de solution de traitement mis en contact avec l'échantillon.

## Revendications

1. Support (6) pour un échantillon d'espèces biologiques recueilli sur un dispositif de prélèvement (1), le dispositif comprenant :
- une tige de préhension (2);
- un élément de capture (3) présentant une surface de capture (3a) pour recevoir l'échantillon, l'élément de capture étant assemblé à la tige de préhension (2) pour former une portion distale (4) du dispositif de prélèvement (1);
le support (6) présentant :
- une face principale (6a) plane délimitée par au moins un côté ;
- un évidemment débouchant sur le côté du support (6) dans lequel peut être inséré le dispositif de prélèvement (1), l'évidemment consistant en une rainure (7) formée sur la face principale (6a) du support (6) pour recevoir la portion distale (4) du dispositif de prélèvement (1), la rainure (7) étant configurée pour coopérer avec la partie distale (4) afin de la maintenir fixement sur ou dans le support (6) ;
le support (6) étant **caractérisé en ce que** il comprend un organe de fixation d'une paroi (8a) sur la face principale (6a) du support (6) pour former un réservoir (8) au-dessus d'une partie au moins de la rainure (7).

2. Support (6) selon la revendication précédente dans lequel la rainure (7) présente une largeur (l1) mesurée au niveau de la face principale (6a) du support (6) strictement inférieure à sa largeur maximale (l2) selon sa profondeur de manière à maintenir la partie distale (4) du dispositif de prélèvement (1) sur ou dans le support (6) lorsque celui-ci est logé dans la rainure (7).

3. Support (6) selon l'une des revendications précédentes présentant une pluralité de rainures (7).

4. Support (6) selon la revendication précédente dans lequel les rainures (7) sont parallèles entre elles.

5. Support (6) selon l'une des revendications précédentes constitué d'un matériau transparent.

6. Support (6) selon la revendication précédente muni sur sa face principale (6a) d'une couche conductrice.

7. Support (6) selon une des revendications 1 à 4 constitué d'une plaque métallique.

8. Support (6) selon l'une des revendications précédentes dans lequel la profondeur (P) de la rainure (7) est telle que la surface de capture (3a) affleure la face principale (6a) du support lorsque la portion distale (4) du dispositif de prélèvement (1) est logée dans la rainure (7).

9. Support (6) selon l'une des revendications précédentes dans lequel les dimensions de la rainure (7) sont ajustées aux dimensions de la portion distale (4) pour former une liaison étanche.

10. Kit de traitement d'un échantillon d'espèces biologiques comprenant un support (6) selon la revendication précédente et une paroi (8a) d'un réservoir (8).

11. Kit de traitement selon la revendication précédente comprenant de plus un capot amovible du réservoir (8).

12. Kit de traitement selon l'une des deux revendications précédentes dans lequel la paroi (8a) du réservoir (8) est formée d'une matière opaque.

13. Kit de traitement selon l'une des revendications 10 à 12 dans lequel le réservoir (8) comprend une pluralité de compartiments étanches.

14. Système d'insertion (10) d'un dispositif de prélèvement (1) dans un support (6), comprenant :
- un support (6) selon l'une quelconque des revendications 1 à 9,
- un premier élément (11) pour placer fixement le support (6) dans un plan d'assemblage ;
- un second élément (12) pour placer fixement dans le plan d'assemblage le dispositif de prélèvement (1), le premier élément (11) et le deuxième élément (12) étant disposé l'un par rapport à l'autre pour que la tige de préhension (2) soit placée en vis-à-vis et aligné à la rainure (7) du support (6) ;
- un dispositif d'entraînement pour déplacer le premier élément (11) vis-à-vis du second élément (12) pour les rapprocher l'un de l'autre et loger la portion distale (4) du dispositif de prélèvement (1) dans la rainure (7).

15. Système d'insertion (11) selon la revendication précédente comprenant un dispositif de coupe pour désolidariser la portion distale (4) du reste de la tige de préhension (2).

## Patentansprüche

1. Träger (6) für eine Probe einer biologischen Spezies, die auf einer Probenahmevorrichtung (1) gesammelt wird, wobei die Vorrichtung umfasst:
- einen Greifstab (2);
- ein Erfassungselement (3), das eine Erfassungsoberfläche (3a) zum Aufnehmen der Probe aufweist, wobei das Erfassungselement zum Bilden eines distalen Abschnitts (4) der Probenahmevorrichtung (1) mit dem Greifstab (2) montiert wird;
wobei der Träger (6) aufweist:
- eine flache Hauptfläche (6a), die von mindestens einer Seite abgegrenzt wird;
- eine Aussparung, die auf der Seite des Trägers (6) mündet und in die die Probenahmevorrichtung (1) eingeführt werden kann, wobei die Aussparung aus einer Nut (7), die auf der Hauptfläche (6a) des Trägers (6) ausgebildet ist, zum Aufnehmen des distalen Abschnitts (4) der Probenahmevorrichtung (1) besteht, wobei die Nut (7) zum Zusammenwirken mit dem distalen Teil (4) konfiguriert ist, um diesen fest auf oder in dem Träger (6) zu halten;
wobei der Träger (6) **dadurch gekennzeichnet ist, dass** er ein Organ zur Befestigung einer Wand (8a) auf der Hauptfläche (6a) des Trägers (6) zum Bilden eines Reservoirs (8) über mindestens einem Teil der Nut (7) umfasst.

2. Träger (6) nach dem vorhergehenden Anspruch, wobei die Nut (7) eine Breite (11) aufweist, die auf der Ebene der Hauptfläche (6a) des Trägers (6) gemessen wird und die strikt kleiner als ihre maximale Breite (12) gemäß ihrer Tiefe ist, um den distalen Teil (4) der Probenahmevorrichtung (1) auf oder in dem Träger (6) zu halten, wenn diese in der Nut (7) untergebracht ist.

3. Träger (6) nach einem der vorhergehenden Ansprüche, der mehrere Nuten (7) aufweist.

4. Träger (6) nach dem vorhergehenden Anspruch, wobei die Nuten (7) parallel zueinander sind.

5. Träger (6) nach einem der vorhergehenden Ansprüche, der aus einem transparenten Material besteht.

6. Träger (6) nach dem vorhergehenden Anspruch, der auf seiner Hauptfläche (6a) mit einer leitfähigen Schicht versehen ist.

7. Träger (6) nach einem der Ansprüche 1 bis 4, der aus einer Metallplatte besteht.

8. Träger (6) nach einem der vorhergehenden Ansprüche, wobei die Tiefe (P) der Nut (7) derart ist, dass die Erfassungsoberfläche (3a) mit der Hauptfläche (6a) des Trägers bündig liegt, wenn der distale Abschnitt (4) der Probenahmevorrichtung (1) in der Nut (7) untergebracht ist.

9. Träger (6) nach einem der vorhergehenden Ansprüche, wobei die Abmessungen der Nut (7) auf die Abmessungen des distalen Abschnitts (4) zum Bilden einer dichten Verbindung abgestimmt sind.

10. Kit zur Verarbeitung einer Probe einer biologischen Spezies, umfassend einen Träger (6) nach dem vorhergehenden Anspruch und eine Wand (8a) eines Reservoirs (8).

11. Verarbeitungskit nach dem vorhergehenden Anspruch, weiterhin umfassend eine abnehmbare Abdeckung des Reservoirs (8).

12. Verarbeitungskit nach einem der zwei vorhergehenden Ansprüche, wobei die Wand (8a) des Reservoirs (8) aus einem opaken Material ausgebildet ist.

13. Verarbeitungskit nach einem der Ansprüche 10 bis 12, wobei das Reservoir (8) mehrere dichte Kammern umfasst.

14. System (10) zum Einführen einer Probenahmevorrichtung (1) in einen Träger (6), umfassend:
- einen Träger (6) nach einem der Ansprüche 1 bis 9,
- ein erstes Element (11) zum festen Anordnen des Trägers (6) in einer Montageebene;
- ein zweites Element (12) zum festen Anordnen der Probenahmevorrichtung (1) in der Montageebene, wobei das erste Element (11) und das zweite Element (12) in Bezug zueinander eingerichtet werden, damit der Greifstab (2) gegenüber der Nut (7) des Trägers (6) und auf diese ausgerichtet angeordnet wird;
- eine Antriebsvorrichtung zum Bewegen des ersten Elements (11) gegenüber dem zweiten Element (12), um diese einander anzunähern und den distalen Abschnitt (4) der Probenahmevorrichtung (1) in der Nut (7) unterzubringen.

15. Einführvorrichtung (11) nach dem vorhergehenden Anspruch, umfassend eine Schneidvorrichtung zum Trennen des distalen Abschnitts (4) von dem Rest des Greifstabs (2).

## Claims

1. Support (6) for a biological species sample collected on a sampling device (1), the device comprising:
- a gripping rod (2),
- a capture element (3) having a capture surface (3a) for receiving the sample, the capture element being assembled with the gripping rod (2) in order to form a distal portion (4) of the sampling device (1);
the support (6) having:
- a flat main face (6a) delimited by at least one side;
- a recess opening out onto the side of the support (6) into which the sampling device (1) can be inserted, the recess consisting of a groove (7) formed on the main face (6a) of the support (6) in order to receive the distal portion (4) of the sampling device (1), the groove (7) being configured to cooperate with the distal portion (4) in order to fixedly hold it on or in the support (6);
the support (6) being **characterised in that** it comprises a member for attaching a wall (8a) onto the main face (6a) of the support (6) to form a reservoir (8) above at least part of the groove (7).

2. Support (6) according to the preceding claim, wherein the groove (7) has a width (11) measured at the main face (6a) of the support (6) that is strictly less than the maximum width (12) thereof depending on the depth thereof so as to hold the distal part (4) of the sampling device (1) on or in the support (6) when the latter is lodged inside the groove (7).

3. Support (6) according to one of the preceding claims having a plurality of grooves (7).

4. Support (6) according to the preceding claim, wherein the grooves (7) are parallel to one another.

5. Support (6) according to one of the preceding claims made of a transparent material.

6. Support (6) according to the preceding claim provided, on the main face (6a) thereof, with a conductor layer.

7. Support (6) according to one of claims 1 to 4 made of a metal plate.

8. Support (6) according to one of the preceding claims, wherein the depth (P) of the groove (7) is such that the capture surface (3a) is flush with the main face (6a) of the support when the distal portion (4) of the sampling device (1) is lodged in the groove (7).

9. Support (6) according to one of the preceding claims, wherein the dimensions of the groove (7) are adjusted to the dimensions of the distal portion (4) to form a sealing connection.

10. Kit for processing a biological species sample comprising a support (6) according to the preceding claim and a wall (8a) of a reservoir (8).

11. Processing kit according to the preceding claim, further comprising a removable cover for the reservoir (8).

12. Processing kit according to one of the preceding two claims, wherein the wall (8a) of the reservoir (8) is made of an opaque material.

13. Processing kit according to one of claims 10 to 12, wherein the reservoir (8) comprises a plurality of sealed compartments.

14. System (10) for inserting a sampling device (1) into a support (6), comprising
- a support (6) according to any one of claims 1 to 9,
- a first element (11) for fixedly positioning the support (6) in an assembly plane;
- a second element (12) for fixedly positioning the sampling device (1) in the assembly plane, the first element (11) and the second element (12) being disposed relative to one another such that the gripping rod (2) is positioned facing and aligned with the groove (7) of the support (6);
- a driving device for moving the first element (11) relative to the second element (12) to move them closer to one another and lodge the distal portion (4) of the sampling device (1) inside the groove (7).

15. Insertion system (11) according to the preceding claim, comprising a cutting device for separating the distal portion (4) from the rest of the gripping rod (2).
